# FASCICULE DE BREVET EUROPEEN

(11) **EP 1 294 303 B1**
(45) Date de publication et mention de la délivrance du brevet: **12.09.2007**
(21) Numéro de dépôt: 01911807.4
(22) Date de dépôt: 26.02.2001
(51) Int. Cl.: A61C 9/00

(54) **MODELE ET PROCEDE POUR LA PRISE D'EMPREINTE TRIDIMENSIONNELLE D'UNE ZONE D'ARCADE DENTAIRE**
MODELL UND VERFAHREN ZUR DREIDIMENSIONALEN ABDRUCKNAHME EINES KIEFERBEREICHS
MODEL AND METHOD FOR TAKING A THREE-DIMENSIONAL IMPRESSION OF A DENTAL ARCH REGION

(30) Priorité: 25.02.2000 FR 0002423
(43) Date de publication de la demande: 26.03.2003
(73) Titulaire: Cynovad, Inc., St Laurent, Quebec H4S 1V9 (CA)
(72) Inventeur: PEROT, Jean-Marc, F-38200 Vienne (FR)
(74) Mandataire: Maureau, Philippe
(86) Numéro de dépôt international: PCT/FR2001/000555
(87) Numéro de publication internationale: WO 2001/064128

(56) Documents cités:
- EP-A- 0 913 130
- WO-A-98/52491
- US-A- 3 704 519
- US-A- 5 466 152
- US-A- 5 938 446

## Description

La présente invention a pour objet un modèle pour la prise d'empreinte tridimensionnelle d'une zone d'arcade dentaire destinée à recevoir une prothèse dentaire. De façon plus spécifique, l'invention concerne un modèle permettant une prise d'empreinte tridimensionnelle qui est ensuite numérisée en vue de réaliser par calcul la détermination de la forme d'une prothèse, et ensuite d'usiner cette prothèse à l'aide d'une machine-outil à commande numérique.

Pour réaliser une prothèse dentaire, telle qu'une couronne ou un bridge, le chirurgien-dentiste commence par préparer la ou les dents qui serviront de support à la prothèse, en taillant ces dents et en formant ce que l'on appelle un moignon ou des moignons.

Il est ensuite procédé à une prise d'empreinte, à l'aide d'un matériau souple, de la totalité de la mâchoire considérée, ou seulement d'une partie de l'arcade de cette mâchoire contenant la zone dans laquelle la prothèse doit être implantée. Il est également procédé à une prise d'empreinte à l'aide d'un matériau souple, et en procédant éventuellement à un mordu, de l'autre mâchoire ou une zone d'arcade de l'autre mâchoire, directement antagoniste à la zone devant recevoir la prothèse. A partir de ces moulages réalisés dans des matériaux souples, un prothésiste réalise un modèle reproduisant la gencive, les dents et les moignons préparés, par coulée d'un matériau à base de plâtre dans les empreintes précédemment obtenues. Il est procédé à la coulée d'un socle à l'aide d'un matériau à base de plâtre. Des pions, qui ont été inclus dans le moulage et qui dépassent de celui-ci, pénètrent dans le socle. Ces pions sont traités de façon à pouvoir être sortis et remis en place dans le socle.

Le prothésiste découpe ensuite la zone devant recevoir la prothèse, qui comporte un moignon dans le cas d'une couronne, ou deux moignons séparés par une cavité dans le cas d'un bridge. Cette séparation est faite par un sciage de l'embase du moulage, sensiblement transversalement à l'arcade.

Dans la technique traditionnelle de réalisation d'une prothèse, le prothésiste retire la partie du moulage destinée à recevoir la prothèse, et construit une prothèse sur cette partie. Au fur et à mesure de la construction, il peut repositionner cette partie du moulage sur le modèle, pour vérifier la cohérence de la construction de la prothèse avec les représentations des dents voisines ainsi que des dents adjacentes lorsqu'il positionne en vis-à-vis les modèles des deux mâchoires.

Dans le cas de la réalisation d'une prothèse mettant en oeuvre une saisie tridimensionnelle de forme à l'aide d'un palpeur mécanique, ou à l'aide d'un capteur optique, en vue de traiter la saisie obtenue pour construire par calcul la prothèse avant de procéder à son usinage, il se pose le problème de réaliser une prise d'empreinte dans des zones peu accessibles, tant d'un point de vue mécanique que d'un point de vue optique, par exemple les zones situées entre les dents. C'est ainsi notamment qu'il peut être difficile d'obtenir la saisie de la forme de la paroi des dents qui sont tournées du côté du moignon, ou la forme de certaines zones du moignon ou des dents adjacentes, dont la position ne peut être atteinte par système optique.

Les documents WO 98/52491, US 3 704 519 et US 5 466 152 décrivent un modèle pour la prise d'empreinte tridimensionnelle d'une zone d'arcade, comprenant un moulage de l'arcade considérée, monté sur un socle (5), la partie du moulage devant recevoir la prothèse étant dissociée du reste du moulage et montée de façon amovible et repositionnable de façon précise sur le socle par l'intermédiaire de pions, dans lequel chaque empreinte de dent ou chaque zone de l'arcade contenant une empreinte de dent, adjacente à l'une des extrémités de la partie destinée à recevoir la prothèse est également dissociée, d'une part, de la partie du moulage devant recevoir la prothèse et, d'autre part, de la zone adjacente appartenant au reste du moulage, et est montée de façon amovible et repositionnable de façon précise sur le socle par l'intermédiaire de pions.

Le document US - A - 5 938 446 décrit un procédé pour la prise d'empreinte tridimensionnelle d'une zone d'arcade, avec mise en oeuvre d'un modèle découpé en plusieurs parties amovibles, ledit procédé connu du document D4 comprenant les étapes suivantes:
- positionner le modèle vis-à-vis du dispositif de saisie de la forme de la zone d'arcade destinée à recevoir la prothèse,
- isoler l'une après l'autre chaque partie amovible du modèle des parties amovibles voisines qui pourraient gêner la saisie de forme, et saisir séparément l'une après l'autre la forme de chaque partie ainsi isolée, et
- reconstituer la forme de l'ensemble de la zone de l'arcade à partir des différentes saisies (cf. D4, col. 4, lignes 17-28).

Le but de l'invention est de fournir un procédé pour la prise d'empreinte tridimensionnelle d'une zone d'arcade dentaire, mettant en oeuvre un modèle du type précité, assurant une accessibilité complète de toutes les faces d'un moulage, dans la zone de réception d'une prothèse, sans nécessiter un travail supplémentaire de la part du laboratoire de prothèses, et en mettant en oeuvre une technique qui est parfaitement maîtrisée par les laboratoires de prothèses.

A cet effet, l'invention concerne un procédé pour la prise d'empreinte tridimensionnelle d'une zone d'arcade, avec mise en oeuvre d'un modèle, comprenant un moulage (2) de l'arcade considérée, monté sur un socle (5), la partie du moulage devant recevoir la prothèse étant dissociée du reste du moulage et montée de façon amovible et repositionnable de façon précise sur le socle par l'intermédiaire de pions, dans lequel chaque empreinte de dent ou chaque zone de l'arcade contenant une empreinte de dent, adjacente à l'une des extrémités de la partie (3) destinée à recevoir la prothèse est également dissociée (6, 8, 9), d'une part, de la partie (3) du moulage devant recevoir la prothèse et, d'autre part, de la zone adjacente appartenant au reste du moulage, et est montée de façon amovible et repositionnable de façon précise sur le socle par l'intermédiaire de pions, caractérisé en ce qu'il consiste :
- à positionner le modèle complet (2) vis-à-vis du dispositif de saisie de la forme de la zone d'arcade destinée à recevoir la prothèse,
- à identifier les emplacements dont la saisie de forme est difficile ou impossible à réaliser en l'état,
- à effectuer, en fonction de l'identification précédente, le retrait des parties du moulage adjacentes à la zone devant recevoir la prothèse avant de réaliser une saisie de la forme de cette zone, et/ou à effectuer le retrait de la partie (3) du moulage de la zone devant recevoir la prothèse, avant de réaliser une saisie de la forme d'au moins une des parties du moulage adjacente à cette zone,
- puis à reconstituer la forme de la zone (10) de l'arcade dont la forme est à obtenir, à partir des différentes saisies de formes effectuées.

L'utilisateur commence par mettre en position dans le système de mesure l'arcade dont il souhaite saisir la forme, dans sa zone d'implantation de la prothèse.

Il est ensuite procédé à l'analyse des difficultés d'accessibilité des différentes zones dont la forme doit être saisie.

Suivant une forme simple de réalisation, la dissociation de la partie du moulage devant recevoir la prothèse et des parties adjacentes du moulage vis-à-vis du reste du moulage est réalisée par sciage sensiblement perpendiculairement à l'arcade, d'une embase solidaire du moulage et reposant sur le socle, sans adhérence avec celui-ci.

Ce modèle est celui qui est utilisé pour réaliser une prise d'empreinte tridimensionnelle soit à l'aide d'un palpeur mécanique, soit à l'aide d'un capteur optique. Les résultats de la mesure sont numérisés avant de subir un traitement permettant de définir le contour de la prothèse.

Suivant une possibilité, le procédé selon l'invention consiste à effectuer une première saisie de la totalité de la zone de l'arcade dont la forme est à obtenir, en vue de l'identification des emplacements dont la saisie de forme est difficile ou impossible à réaliser en l'état.

En fait, l'analyse des difficultés d'accessibilité peut être effectuée de différentes manières : par l'utilisateur qui connaît les limites du système de prise d'empreinte, par le système de prise d'empreinte à l'aide d'un dispositif de détection automatique, tel que vidéo en deux dimensions ou pré-mesure rapide en trois dimensions, ou par l'utilisateur avec l'assistance de moyens d'analyse incorporés dans le système de prise d'empreinte.

A l'issue de cette phase d'analyse, les zones inaccessibles sont identifiées.

Le procédé consiste ensuite à déterminer la stratégie des saisies de formes successives, c'est-à-dire l'ordre dans lequel seront démontées les différentes parties du moulage et effectuées les différentes prises d'empreinte.

La stratégie peut être définie de différentes manières :
- par l'utilisateur qui connaît les limites du système de prise d'empreinte,
- par le système de prise d'empreinte à l'aide d'un algorithme approprié,
- ou par l'utilisateur avec l'assistance de moyens de simulation incorporés dans le système de prise d'empreinte.

Il est ensuite procédé à la prise d'empreinte elle-même par application de la stratégie qui a été définie, c'est-à-dire avec démontage des parties de l'arcade générant une inaccessibilité des parties voisines et mesure des parties ainsi rendues accessibles. Il est procédé ensuite à la remise en place des parties préalablement démontées avec application du même principe jusqu'à l'obtention de la mesure complète.

A cet effet, le procédé selon l'invention, consiste à piloter le dispositif de prise d'empreinte afin que celui-ci :
- informe l'utilisateur de l'attente d'un démontage et/ou d'un remontage d'une partie d'arcade,
- suspende une prise d'empreinte pour permettre le démontage d'une partie d'arcade,
- reprenne la mesure après démontage, et
- reconstitue la forme de la zone de l'arcade dont la forme est à obtenir, à partir des différentes saisies de formes effectuées.

De toute façon, l'invention sera bien comprise à l'aide de la description qui suit, en référence au dessin schématique annexé représentant, en vue de dessus, une forme d'exécution de ce modèle :
Figure 1 est une vue de dessus d'un modèle en plâtre correspondant à une partie de mâchoire ;
Figure 2 en est une vue en perspective avec identification de la zone dont la saisie optique doit être réalisée ;
Figures 3 et 4 en sont deux vues en perspective respectivement au cours de deux phases de la saisie tridimensionnelle de forme.

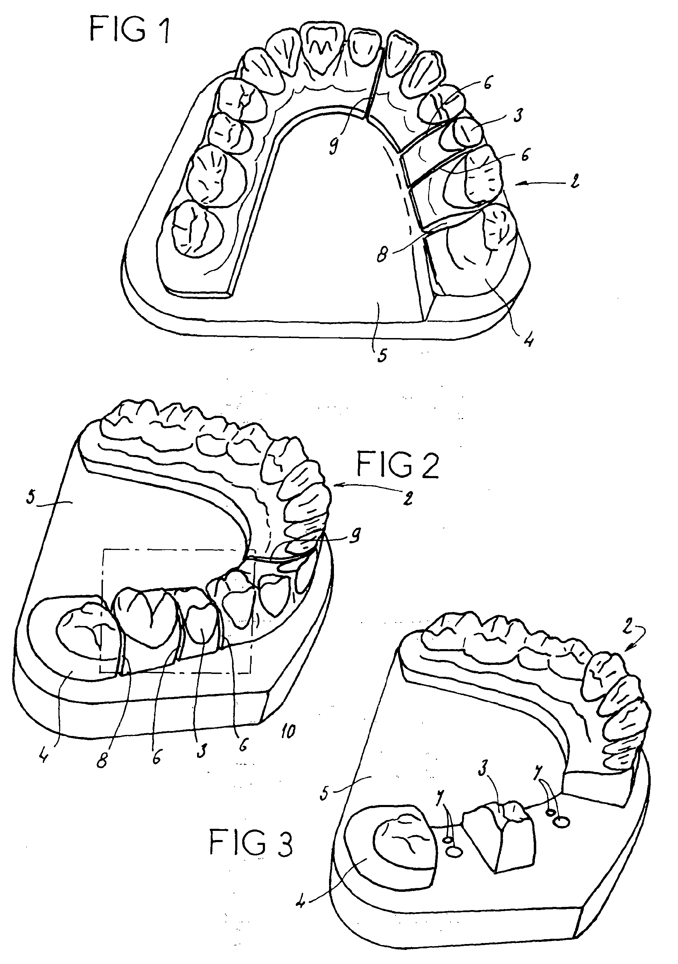

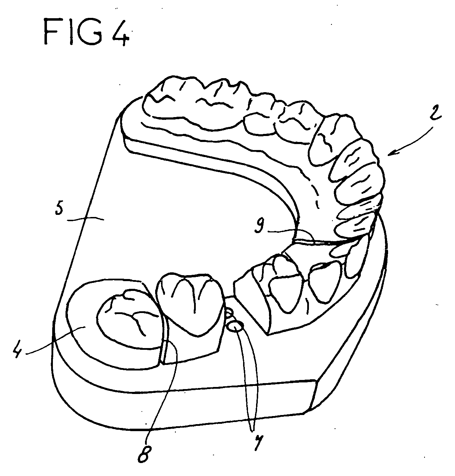

La figure 1 représente un modèle d'une partie de mâchoire, comportant un moulage en plâtre 2 d'un certain nombre de dents, dont un moignon 3 destiné à recevoir une couronne. Le moulage en plâtre comprend une embase 4, qui repose sur un socle 5. Comme montré au dessin, la partie de moulage comportant le moignon 3 est séparée des parties adjacentes par deux fentes 6 sensiblement transversales à l'arcade formée par la mâchoire. L'embase 4 n'étant pas adhérente sur le socle 5, il est possible de retirer la partie comportant le moignon 3, comme cela est montré à la figure 4, cette partie pouvant être repositionnée de façon précise grâce à des pions, non représentés au dessin, engagés dans deux trous 7 ménagés dans le socle. De telles coupures, respectivement 8 et 9, sont ménagées, d'une part, de l'autre côté de la dent adjacente au moignon 3, et située du côté de l'arrière de la mâchoire et, d'autre part, à l'autre extrémité d'un groupe de trois dents situées du côté avant de la mâchoire. Les deux parties délimitées par les fentes 6, 8, d'une part, et 6 et 9, d'autre part, peuvent être retirées indépendamment l'une de l'autre et repositionnées de façon précise à l'aide d'un système de pion, comme tel est le cas pour la partie du moulage comportant le moignon 3.

Il peut être constaté, en analysant la figure 2, que les zones entre le moignon et les deux dents adjacentes sont très étroites, de telle sorte qu'il est difficile, voire impossible, de réaliser une prise d'empreinte correcte des faces du moignon tournées vers les deux dents adjacentes, ainsi que des faces des dents adjacentes tournées vers le moignon.

Il est tout d'abord procédé à l'identification de la zone dont la prise d'empreinte doit être effectuée. Cette zone, désignée par la référence 10, est délimitée par des traits mixtes à la figure 2. Comme montré à la figure 3, il est procédé tout d'abord au démontage des deux groupes de dents situés de part et d'autre du moignon 3, pour procéder à la saisie de la forme du moignon, qui est maintenant parfaitement accessible. Les deux groupes de dents adjacentes au moignon sont remontés, après quoi il est procédé au retrait du moignon, comme montré à la figure 4. Il est alors procédé à la mesure des dents adjacentes et du socle, après quoi il est procédé par calcul à la reconstruction algorithmique de la saisie de forme complète de la zone 10, avec élimination du socle.

Cette saisie étant effectuée, il est possible de procéder à la détermination de la forme de la prothèse, qui est destinée à équiper le moignon 3.

Comme il ressort de ce qui précède, l'invention apporte une grande amélioration à la technique existante en fournissant un modèle de structure simple et mis en oeuvre à l'aide d'un procédé simple également, permettant une saisie tridimensionnelle de toutes les parties du modèle destiné à recevoir une prothèse. La mise en oeuvre de ce modèle et du procédé correspondant sont effectuées en tenant compte des règles de l'art connues dans le domaine de la prothèse dentaire.

## Revendications

1. Procédé pour la prise d'empreinte tridimensionnelle d'une zone d'arcade, avec mise en oeuvre d'un modèle, comprenant un moulage (2) de l'arcade considérée, monté sur un socle (5), la partie du moulage devant recevoir la prothèse étant dissociée du reste du moulage et montée de façon amovible et repositionnable de façon précise sur le socle par l'intermédiaire de pions, dans lequel chaque empreinte de dent ou chaque zone de l'arcade contenant une empreinte de dent, adjacente à l'une des extrémités de la partie (3) destinée à recevoir la prothèse est également dissociée (6, 8, 9), d'une part, de la partie (3) du moulage devant recevoir la prothèse et, d'autre part, de la zone adjacente appartenant au reste du moulage, et est montée de façon amovible et repositionnable de façon précise sur le socle par l'intermédiaire de pions, consistant :
- à positionner le modèle complet (2) vis-à-vis du dispositif de saisie de la forme de la zone d'arcade destinée à recevoir la prothèse,
- à identifier les emplacements dont la saisie de forme est difficile ou impossible à réaliser en l'état,
- à effectuer, en fonction de l'identification précédente, le retrait des parties du moulage adjacentes à la zone devant recevoir la prothèse avant de réaliser une saisie de la forme de cette zone, et/ou à effectuer le retrait de la partie (3) du moulage de la zone devant recevoir la prothèse, avant de réaliser une saisie de la forme d'au moins une des parties du moulage adjacente à cette zone,
- puis à reconstituer la forme de la zone (10) de l'arcade dont la forme est à obtenir, à partir des différentes saisies de formes effectuées.

2. Procédé selon la revendication 1, **caractérisé en ce que** la dissociation de la partie (3) du moulage devant recevoir la prothèse et des parties adjacentes du moulage vis-à-vis du reste du moulage est réalisée par sciage (6, 8, 9) sensiblement perpendiculairement à l'arcade, d'une embase (4) solidaire du moulage et reposant sur le socle (5), sans adhérence avec celui-ci.

3. Procédé selon la revendication 1, **caractérisé en ce qu'**il consiste à effectuer une première saisie de la totalité de la zone de l'arcade dont la forme est à obtenir, en vue de l'identification des emplacements dont la saisie de forme est difficile ou impossible à réaliser en l'état.

4. Procédé selon la revendication 3, **caractérisé en ce qu'**il consiste ensuite à déterminer la stratégie des saisies de formes successives, c'est-à-dire l'ordre dans lequel seront démontées les différentes parties du moulage et effectuées les différentes prises d'empreinte.

5. Procédé selon l'une quelconque des revendications 1 à 4, **caractérisé en ce qu'**il consiste à piloter le dispositif de prise d'empreinte afin que celui-ci :
- informe l'utilisateur de l'attente d'un démontage et/ou d'un remontage d'une partie d'arcade,
- suspende une prise d'empreinte pour permettre le démontage d'une partie d'arcade,
- reprenne la mesure après démontage, et
- reconstitue la forme de la zone (10) de l'arcade dont la forme est à obtenir, à partir des différentes saisies de formes effectuées.

## Claims

1. Method for taking a three-dimensional impression of a dental arch region, using a model that comprises a mould (2) of the dental arch concerned, mounted on a base (5), the part of the mould intended to receive the prosthesis being dissociated from the rest of the mould and being mounted so as to be removable and precisely repositionable on the base by way of pegs, in which each tooth impression or each dental arch region containing a tooth impression, adjacent to one of the ends of the part (3) intended to receive the prosthesis, is also dissociated (6, 8, 9) on the one hand from the part (3) of the mould intended to receive the prosthesis and on the other hand from the adjacent region belonging to the rest of the mould, and is mounted so as to be removable and precisely repositionable on the base by way of pegs, said method consisting in:
- positioning the complete model (2) opposite the device for recording the shape of the dental arch region intended to receive the prosthesis,
- identifying the locations where recording the shape is difficult or impossible in the given circumstances,
- removing, on the basis of the preceding identification, the parts of the mould that are adjacent to the region intended to receive the prosthesis before recording the shape of this region, and/or removing the part (3) of the mould from the region intended to receive the prosthesis before recording the shape of at least one of the parts of the mould adjacent to this region,
- then reconstructing the shape of the dental arch region (10) whose shape is to be obtained, on the basis of the various shapes recorded.

2. Method according to Claim 1, **characterized in that** the dissociation of the part (3) of the mould intended to receive the prosthesis and of the adjacent parts of the mould from the rest of the mould is effected by sawing (6, 8, 9), substantially perpendicular to the dental arch, a platform (4) integral with the mould and resting on the base (5), without adhering to the latter.

3. Method according to Claim 1, **characterized in that** it consists in first recording the whole of the dental arch region whose shape is to be obtained, with a view to identifying locations whose shape is difficult or impossible to record in the given circumstances.

4. Method according to Claim 3, **characterized in that** it then consists in determining the strategy for recording successive shapes, that is to say the order in which the different parts of the mould will be disassembled and the different impressions will be taken.

5. Method according to any one of Claims 1 to 4, **characterized in that** it consists in controlling the impression-taking device in such a way that the latter:
- informs the user of expected disassembly and/or reassembly of a part of the dental arch,
- suspends the taking of an impression in order to permit disassembly of a part of the dental arch,
- resumes the measurement after disassembly, and
- reconstructs the shape of the dental arch region (10) whose shape is to be obtained, on the basis of the various shapes recorded.

## Patentansprüche

1. Verfahren zur Anfertigung eines dreidimensionalen Abdrucks eines Zahnbogenbereichs, unter Verwendung eines Modells, enthaltend einen Abguss (2) des betreffenden Zahnbogens, der auf einer Sockelplatte (5) montiert ist, wobei derjenige Teil des Abgusses, der für die Aufnahme der Prothese bestimmt ist, vom Rest des Abgusses getrennt ist und auf abnehmbare und präzise wiederanbringbare Weise mit Hilfe von Pins aus der Sockelplatte montiert ist, bei dem jeder Zahnabdruck oder jeder Zahnbogenbereich, der einen Zahnabdruck enthält, der an eines der Enden desjenigen Teils (3) angrenzt, der für die Aufnahme der Prothese bestimmt ist, auch einerseits von demjenigen Teil (3) des Abgusses, der für die Aufnahme der Prothese bestimmt ist, und andererseits von dem angrenzenden Bereich, der zum Rest des Abdrucks gehört, getrennt (6, 8, 9) ist und auf abnehmbare und präzise wiederanbringbare Weise mit Hilfe von Pins auf der Sockelplatte montiert ist, das darin besteht:
• das vollständige Modell (2) der Vorrichtung zur Erfassung der Form des Zahnbogenbereichs, der für die Aufnahme der Prothese bestimmt ist, gegenüberliegend anzuordnen,
• diejenigen Stellen zu identifizieren, deren Formerfassung sich in diesem Zustand schwer oder unmöglich realisieren lässt,
• in Abhängigkeit der vorhergehenden Identifizierung diejenigen Teile des Abgusses abzunehmen, die an den Bereich angrenzen, der für die Aufnahme der Prothese bestimmt ist, bevor eine Erfassung der Form dieses Bereichs realisiert wird, und/oder denjenigen Teil (3) des Abgusses des Bereiches abzunehmen, der für die Aufnahme der Prothese bestimmt ist, bevor eine Erfassung der Form zunindest eines der Teile des Abgusses, der an diesen Bereich angrenzt, realisiert wird,
• anschließend die Form desjenigen Bereichs (10) des Zahnbogens, dessen Form zu erhalten ist, ausgehend von den verschiedenen, durchgeführten Formerfassungen nachzubilden.

2. Verfahren nach Anspruch 1, **dadurch gekennzeichnet, dass** das Trennen desjenigen Teils (3) des Abgusses, der für die Aufnahme der Prothese bestimmt ist, und der angrenzenden Teile des Abgusses, die dem Rest des Abgusses gegenüberliegen, **dadurch** erfolgt, dass eine Grundplatte (4), die fest mit dem Abguss fest verbunden ist und auf dem Sockel (5) aufliegt, ohne mit diesem letzten verbunden zu sein, im Wesentlichen senkrecht zu dem Zahnbogen durchgesägt (6, 8, 9) wird.

3. Verfahren nach Anspruch 1, **dadurch gekennzeichnet, dass** es darin besteht, eine erste Erfassung des gesamten Bereichs des Zahnbogens durchzuführen, dessen Form erhalten werden soll, un diejenigen Stellen zu identifizieren, deren Formerfassunc in diesem Zustand schwierig oder unmöglich ist.

4. Verfahren nach Anspruch 3, **dadurch gekennzeichnet, dass** es darin besteht, anschließen die Strategie festzulegen, mit der die nachfolgenden Formerfassungen erfolgen, d. h. die Reihenfolge, in der die unterschiedlichen Teile des Abgusses demontiert werden und die verschiedenen Abdrucknahmen erfolgen.

5. Verfahren nach einem der Ansprüche 1 bis 4,
**dadurch gekennzeichnet, dass** es darin besteht, die Vorrichtung zur Anfertigung des Abdrucks zu steuern, so dass diese:
• der Benutzer darüber zu informiert, dass sie auf die Demontage und/oder Montage eines Zahnbogenbereichs wartet,
• eine Abdrucknahme unterbricht, um die Demontage eines Zahnbogenteils zu ermöglichen,
• nach der Demontage die Maßnahme wiederaufnimmt und
• die Form desjenigen Bereichs (10) des Zahnbogens, dessen Form zu erfassen ist, ausgehend von den verschiedenen, durchgeführten Formerfassungen nachbildet.
